**Europäisches Patentamt**

**European Patent Office**

**Office européen des brevets**

(19)

(11) Numéro de publication: **0 074 914**
**B1**

(12)

# FASCICULE DE BREVET EUROPEEN

(45) Date de publication du fascicule du brevet:
**27.12.84**

(21) Numéro de dépôt: **82420126.3**

(22) Date de dépôt: **06.09.82**

(51) Int. Cl.³: **A 61 N 5/06,** A 61 H 39/00,
B 23 K 26/10

(54) **Appareil pour l'application de traitements thérapeutiques du type de l'acupuncture.**

(30) Priorité: **07.09.81 FR 8117276**

(43) Date de publication de la demande:
**23.03.83 Bulletin 83/12**

(45) Mention de la délivrance du brevet:
**27.12.84 Bulletin 84/52**

(84) Etats contractants désignés:
**BE CH DE GB IT LI NL SE**

(56) Documents cités:
**DE - A - 2 740 969**
**DE - A - 2 826 383**
**FR - A - 2 481 915**
**US - A - 4 180 079**
**US - A - 4 273 127**

(73) Titulaire: **Société Coopérative Ouvrière de Production à
Responsabilité Limitée: ERIEL - Société d'Engineering
et de, Réalisations Electroniques et Industrielles,
F-48230 Chanac (FR)**

(72) Inventeur: **Matton, Jean-François, 3b Touleuses
Pourpres, F-95000 Cergy (FR)**
Inventeur: **Esclapez, Richard, 10, Avenue Ramolfo
Garnier, F-91300 Massy (FR)**

(74) Mandataire: **Ropital-Bonvarlet, Claude et al, Cabinet
BEAU DE LOMENIE 99, Grande rue de la Guillotière,
F-69007 Lyon (FR)**

## Description

La présente invention concerne les appareils permettant de réaliser des traitements thérapeutiques du type de l'acupuncture, sans faire intervenir l'application des aiguilles traditionnelles de cette technique, et elle concerne, plus particulièrement, les appareils mettant en œuvre un rayonnement lumineux.

Des appareils du type ci-dessus sont connus (DE-A N° 2740969) et déjà utilisés pour l'application de traitements thérapeutiques visant des points ou centres nerveux corporels, en application tant à l'homme qu'à l'animal.

De tels appareils sont constitués par un boîtier préhensible contenant un générateur de rayonnement lumineux, généralement du type laser. Un tel générateur est prolongé par un élément de conduction du rayonnement lumineux, le plus souvent constitué par une fibre optique, dont la partie terminale, opposée au générateur, peut être munie d'un embout métallique d'application faisant saillie à l'extérieur du boîtier.

L'utilisation d'un tel appareil consiste à assurer l'application de l'extrémité de l'élément de conduction sur le ou les points ou centres nerveux en vue d'y appliquer le rayonnement lumineux.

De tels appareils peuvent être alimentés par une source d'énergie autonome ou être reliés par un cordon à une source indépendante.

De tels appareils peuvent être considérés comme donnant satisfaction lorsqu'il s'agit de pratiquer un traitement thérapeutique au niveau de points ou centres nerveux qualifiés d'externes, c'est-à-dire directement accessibles, par opposition à ceux situés dans les fosses, cavités ou voies naturelles et dénommés internes.

Par contre, on comprend que de tels appareils ne peuvent être pratiquement utilisés pour atteindre les points ou centres nerveux du second type, car la conformation du boîtier et celle de l'élément de conduction n'autorisent aucune déformation et n'offrent pas une forme suffisamment longue, effilée ou pointue, pour favoriser une pénétration aisée, rapide et indolore dans les fosses, cavités ou voies naturelles.

L'objet de l'invention est de remédier à cet inconvénient en proposant des moyens adaptables aux appareils connus et aptes à rendre de tels appareils utilisables de façon traditionnelle ou pour l'application de traitements thérapeutiques au niveau de points ou centres nerveux situés dans les fosses, cavités ou voies naturelles du corps humain ou animal.

L'objet de l'invention est de fournir des moyens particulièrement simples, fiables et peu onéreux, pour rendre possible, en cas de besoin, l'extension de l'extrémité de l'élément de conduction hors du boîtier en vue de pénétrer et d'atteindre les points ou centres nerveux situés dans les fosses, cavités ou voies naturelles.

Un autre objet de l'invention est de proposer des moyens pouvant être mis en œuvre dans les chaînes de fabrication actuelle des appareils du type connu, sans apporter de modification notable aux constituants principaux.

Un autre objet de l'invention est de fournir des moyens qui peuvent être associés à une commande directement manuelle ou motorisée.

Pour atteindre les buts ci-dessus, l'objet de l'invention comprend:

— un boîtier, contenant un ensemble fonctionnel formé d'un générateur de rayonnement lumineux et d'une fibre optique, dont la partie terminale opposée au générateur fait saillie du boîtier,

— des moyens d'entraînement en coulissement en courses d'extension et/ou de rétraction de la fibre par rapport au boîtier,

— des moyens de support et de guidage en déplacement.

Diverses autres caractéristiques de l'invention ressortent de la description ci-dessous, faite en référence aux dessins annexés qui montrent, à titre d'exemples non limitatifs, des formes de réalisation de l'invention.

La fig. 1 est une élévation latérale, en partie arrachée, d'un appareil conforme à l'invention.

La fig. 2 est une vue latérale, à plus grande échelle, illustrant l'un des éléments constitutifs de l'objet de l'invention.

La fig. 3 est une coupe transversale, prise à plus grande échelle, selon la ligne III-III de la fig. 1.

La fig. 4 et la fig. 5 sont des coupes longitudinales partielles, montrant plus en détail certains des moyens de l'invention.

La fig. 6 est une élévation latérale partielle montrant, à plus grande échelle, une variante de réalisation de l'un des éléments constitutifs de l'objet de l'invention.

La fig. 7 est une coupe-élévation, illustrant, à échelle différente, une autre forme de réalisation de certains des éléments constitutifs de l'invention.

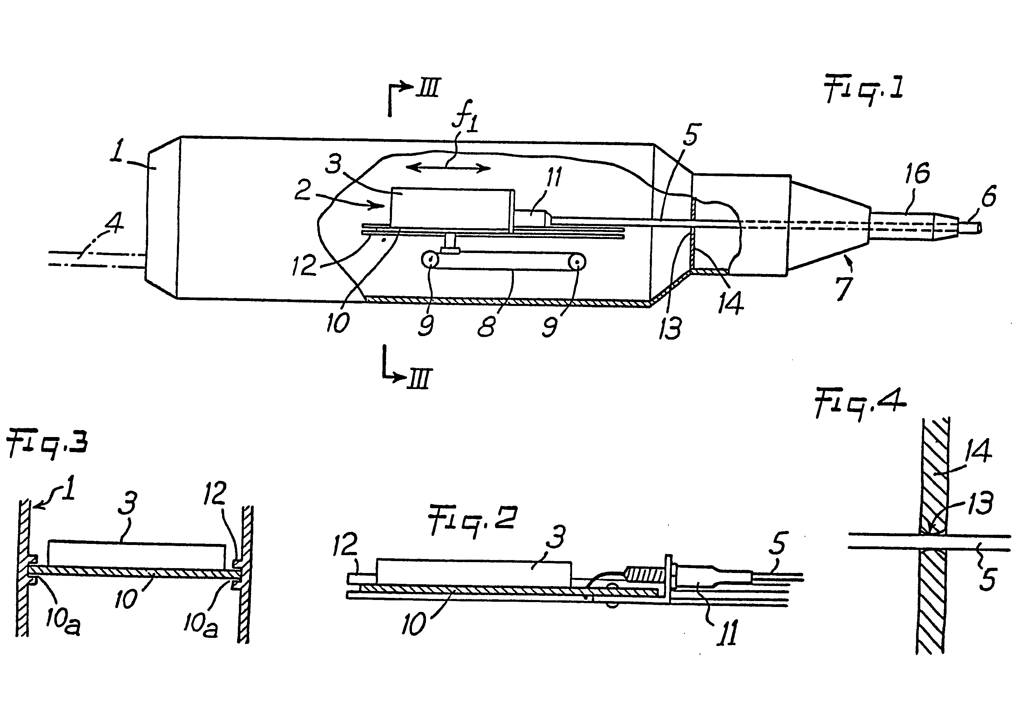

Selon la fig. 1, l'appareil pour l'application de traitements thérapeutiques du type de l'acupuncture comprend un boîtier 1, en toute matière appropriée, de préférence constitué en plusieurs coquilles démontables, assemblées relativement par des moyens d'encliquetage ou par des vis. Le boîtier 1 contient un ensemble fonctionnel 2, comprenant un générateur 3 de rayonnement lumineux pouvant être alimenté par une source interne au boîtier ou éventuellement externe par un cordon d'alimentation 4. L'ensemble 2 comprend également un élément de conduction du rayonnement lumineux 5, constitué par une fibre optique dont la partie terminale, opposée au générateur 3, est par exemple associée à un embout métallique 6 faisant, au moins en partie, saillie en bout d'un canon 7 formé par le boîtier 1.

Selon l'invention, une partie au moins de l'ensemble 2 est associée à des moyens d'entraînement en coulissement en courses d'extension et/ou de rétraction de l'embout métallique 6 par rapport au canon 7. Selon l'exemple illustré par les fig. 1, 2 et 3, ces moyens d'entraînement comprennent un élément souple 8 établi en forme de boucle fermée. L'élément souple 8 peut être un fil, une sangle, une bande crantée ou non, une chaînette ou tout autre élément analogue. L'élément souple 8 coopère avec deux galets de renvoi 9 qui peuvent être constitués sous la forme de roues ou de pignons en complémentarité de la structure propre de l'élément 8. Les roues 9 représentent des organes de renvoi et sont montées sur des axes portés par le boîtier 1. L'une des roues 9 constitue simultanément un organe d'entraînement en défilement dans un sens ou dans l'autre de l'élément 8 et se trouve associée à cette fin à un organe d'entraînement en rotation (non représenté). L'organe d'entraînement en rotation peut être constitué par une commande manuelle ou une commande motorisée incluant, par exemple, un moteur électrique ou pneumatique, dont la mise en fonctionnement et le sens de rotation sont déterminés par un ou plusieurs boutons de commande portés de préférence par le boîtier 1.

L'élément souple 8 est fixé au générateur 3 qui est, de préférence, constitué par une platine 10 supportant un circuit générateur électronique capable de commander le fonctionnement d'une diode laser 11 portée par la platine 10. La diode laser 11 est prolongée par la fibre optique 5 traversant le canon 7.

Les moyens d'entraînement ci-dessus permettent, par conséquent, de commander le déplacement linéaire alternatif du générateur 3 dans l'un ou l'autre sens de la flèche $f_1$, parallèlement à l'axe longitudinal du boîtier 1. L'amplitude de déplacement est déterminée par les caractéristiques constructives des moyens d'entraînement et notamment, dans le cas présent, par l'écartement ou la mesure séparant les galets 9.

Selon une réalisation de l'invention, les moyens d'entraînement en coulissement sont associés à des moyens de support et de guidage en déplacement de l'ensemble 2. Ces moyens font tout d'abord intervenir des organes de support et de guidage 12 pour le générateur 3. Ces organes 12 peuvent être constitués par des glissières ou des guides de coulissement et de support prévus entre le boîtier 1 et la platine 10. De préférence, ces organes 12 sont constitués directement par des rainures formées par les parois internes du boîtier 1 et dans lesquelles sont introduits, avec possibilité de coulissement, les bords 10a de la platine 10 supportant le générateur 3. Les organes 12 sont établis selon une direction parallèle au sens de la flèche $f_1$.

Les moyens de support et de guidage en déplacement font également intervenir des organes de support et de guidage de la fibre optique 5. La fig. 4 montre que ces organes comprennent, tout d'abord, un palier lisse 13, ménagé dans une cloison 14, prévue à l'intérieur du boîtier 1, par exemple dans la zone intermédiaire comprise entre ce dernier et le canon 7. Le palier 13 est conformé de manière à offrir une surface de frottement la plus faible possible, de façon à constituer à la fois un élément de support s'opposant à la flexion de la fibre 5 et une lunette de guidage en coulissement de cette dernière.

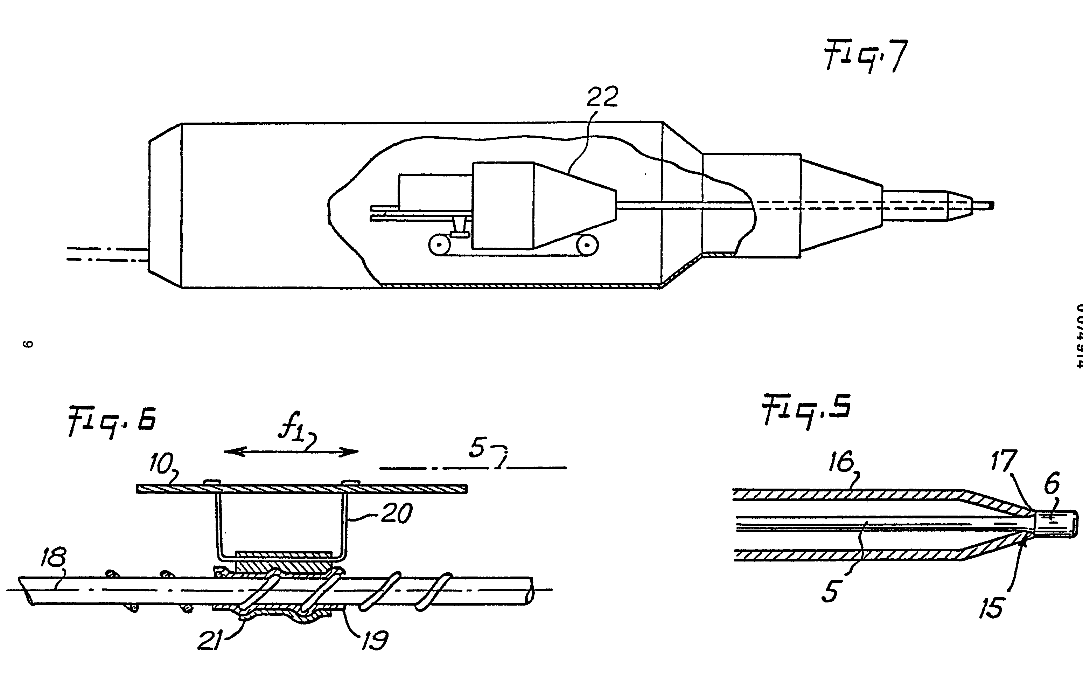

Ces moyens font également intervenir, comme illustré par la fig. 5, un second palier lisse 15 qui est ménagé en bout d'un guide tubulaire 16 formé dans le prolongement du canon 7. Le guide 16 assume, par le palier 15, une fonction de support et de guidage en coulissement de la partie terminale de la fibre optique 5. En outre, le guide tubulaire 16 possède une face transversale plane 17 servant d'appui et de butée à l'embout métallique 6 équipant la partie terminale extrême de la fibre optique 5.

Selon une autre réalisation de l'invention, la fibre optique possède une longueur telle que, dans la position de recul maximal de l'ensemble 2, telle qu'illustrée par la fig. 1, l'embout 6 est en butée ou sensiblement en butée contre la face d'appui 17 du guide 16.

Dans cet état, l'appareil peut être utilisé de façon traditionnelle, c'est-à-dire en assurant l'application de l'extrémité de l'élément de conduction 5 sur des points ou des centres nerveux externes, c'est-à-dire directement accessibles au niveau de l'épiderme externe du corps humain ou animal. Une telle utilisation peut être menée à bien correctement, de façon traditionnelle et avec précision, étant donné que l'extrémité de la fibre 5 est maintenue contre toute déviation axiale, notamment par la combinaison des moyens 15 et 17 coopérant respectivement avec la fibre optique 5 et avec l'embout 6.

Lorsqu'un traitement thérapeutique du type de l'acupuncture doit être appliqué sur des points ou centres nerveux internes, c'est-à-dire accessibles uniquement à partir de fosses, cavités ou voies naturelles, le praticien agit sur les moyens d'entraînement en coulissement, en l'occurrence la roue menante 9, de manière à provoquer le déplacement du générateur 3 en direction du canon 7 à partir de la position de référence illustrée par la fig. 1. La platine 10 coulisse alors en étant supportée et guidée par les organes 12 et provoque le déplacement, dans le sens correspondant, de la fibre optique 5 qui est supportée et guidée par les paliers lisses 13 et 15. Il en résulte une course d'extension de la fibre optique 5 par rapport au guide tubulaire 16 et un transfert de l'embout d'application 6 à l'extérieur et dans le prolongement axial du guide 16.

Le coulissement de l'ensemble fonctionnel 2 peut être commandé sur tout ou partie de la longueur de la course maximale utile en fonction de la profondeur locale des points à atteindre.

Dans cet état, la souplesse relative de la fibre optique ainsi que la faible section de cette dernière permettent une introduction dans les fosses, cavités ou voies naturelles, avec déformation par flexion élastique possible.

Après traitement, le praticien commande dans le sens inverse les moyens d'entraînement en déplacement pour ramener l'ensemble fonctionnel 2 dans la position initiale de référence, comme illustré par la fig. 1.

Ainsi que cela ressort de ce qui précède, les moyens de l'invention permettent de réaliser, de façon simple, fiable, pratique et rapide, l'extension et/ou la rétraction de l'embout d'application par rapport au boîtier de l'appareil. Cela rend possible une utilisation pour l'application de traitements sur des points ou centres nerveux externes ou internes, indifféremment.

Les moyens généraux mis en œuvre sont robustes et aisément adaptables aux chaînes de fabrication de tels appareils, tant de montage que de fabrication.

Les moyens mis en œuvre permettent en outre d'obtenir une grande qualité de fonctionnement souple et précis, sans aucun risque de détérioration de la fibre optique ou de la liaison entre cette dernière et la diode laser 11, étant donné les moyens de support et de guidage 13 et 15 mis en œuvre.

Une précision de commande particulièrement simple de la course d'extension et/ou de rétraction peut être obtenue en faisant intervenir, entre les moyens d'entraînement et l'organe de commande utilisé, des éléments de démultiplication.

La fig. 6 montre une variante de réalisation selon laquelle les moyens d'entraînement en coulissement en courses d'extension et/ou de rétraction sont constitués par une vis sans fin 18 possédant un pas de grande amplitude pouvant être formé par un filet usiné ou rapporté en étant éventuellement constitué par un cordon soudé. La vis sans fin 18 est portée par le boîtier parallèlement à la direction de coulissement selon la flèche f₁ et se trouve associée à un organe de commande manuelle ou motorisée.

La vis sans fin 18 coopère avec un écrou 19 qui est porté par la platine 10 du générateur 3. De préférence, l'écrou 19 est constitué par un segment de gaine thermorétractable enveloppant la vis 18. La liaison entre l'écrou 19 et la platine 10 peut être établie par l'intermédiaire d'un pontet ou d'un étrier 20 associé à une bride 21 pouvant être aussi réalisée en une matière plastique thermorétractable entourant partiellement l'écrou 19.

Des moyens techniques équivalents à ceux décrits ci-dessus peuvent être mis en œuvre pour assumer la fonction d'entraînement en déplacement en course d'extension et/ou de rétraction.

La fig. 7 montre une variante de réalisation de certains des moyens de guidage et de support. Selon cette variante, la platine 10 du générateur 3 supporte une buse 22 entourant la partie frontale et, notamment, la diode laser et s'étendant en direction du canon 7 pour représenter un organe de maintien de la fibre 5. La buse 22 possède une conformation extérieure exactement complémentaire au volume interne délimité par le canon 7.

Dans la position de rétraction maximale, le maintien contre la flexion de la fibre 5 est assuré par le palier 15 et par l'extrémité de la buse 22. Lors de l'amenée en course d'extension maximale, la buse 22 s'engage et se trouve centrée à l'intérieur du canon 7 où elle maintient la fibre 5, par ailleurs soutenue par le palier 15.

Dans ce qui précède, il est indiqué que, selon l'invention, les moyens d'entraînement en coulissement en courses d'extension et/ou de rétraction intéressent tout l'ensemble fonctionnel. Il peut être prévu, selon une variante de réalisation, de monter, à l'intérieur du boîtier 1, des moyens d'entraînement en coulissement intéressant uniquement la fibre optique 5. Ces moyens peuvent alors comprendre une platine fixe 10 occupant une position axiale fixe et une fibre optique 5 souple, possédant une longueur nettement supérieure à celle comprise entre la position fixe du générateur 3 et l'extrémité ou la face 17 du guide 16. La longueur excédentaire de la fibre optique 5 est prise en charge par un organe mobile associé à un organe de commande externe. L'organe mobile peut être constitué par un tambour d'enroulement ou une galette ou encore par une pince coulissante chargée de commander le déroulement ou la déformation d'une boucle tampon formée par la fibre optique 5. De tels moyens sont mis en œuvre en coopération avec les moyens de support et de guidage tels que les paliers 10, 13 et 15.

## Revendications

1. Appareil pour l'application de traitements thérapeutiques du type de l'acupuncture comprenant:
   — un boîtier (1) contenant un ensemble fonctionnel (2) formé d'un générateur de rayonnement lumineux (3) et d'une fibre optique (5) dont la partie terminale opposée au générateur fait saillie du boîtier,
   — des moyens (8-9) d'entraînement en coulissement en courses d'extension et/ou de rétraction de la fibre par rapport au boîtier,
   — des moyens (12-13-15) de support et de guidage en déplacement.

2. Appareil selon la revendication 1, caractérisé en ce que les moyens d'entraînement (8), de support (12) et de guidage (13-15), coopèrent avec une fibre optique souple (5) s'étendant à partir du générateur (3) en présentant une longueur supérieure à la distance séparant ledit générateur d'une butée (17) formée par le boîtier pour l'appui de l'embout (16) en état de rétraction de la fibre.

3. Appareil selon la revendication 2, caractérisé en ce que les moyens d'entraînement sont formés par un organe mobile prenant en charge la longueur excédentaire de la fibre (5) et associé à un organe de commande.

4. Appareil selon la revendication 2, caractérisé en ce que les moyens de support et de guidage comprennent au moins un palier (13 ou 15) présenté par au moins un élément constitutif du boîtier.

5. Appareil selon la revendication 1, caractérisé en ce que les moyens d'entraînement, de support et de guidage coopèrent avec l'ensemble actif.

6. Appareil selon la revendication 5, caractérisé en ce que les moyens d'entraînement (8), associés à un organe de commande, provoquent le déplacement du générateur (3) entraînant la fibre (5) en coulissement et en ce que les moyens de support et de guidage comprennent:
   — des guides (12-10a) de coulissement prévus entre le boîtier (1) et le générateur (3),

— au moins un palier (13-15) présenté par au moins un élément du boîtier pour le support et le guidage de la fibre.

7. Appareil selon l'une des revendications 4 ou 6, caractérisé en ce que les moyens de support et de guidage comprennent un palier lisse (13) présenté par une paroi transversale (14) du boîtier et un palier lisse (15) délimité par un canon de guidage (7) prolongeant le boîtier.

8. Appareil selon l'une des revendications 4 ou 6, caractérisé en ce que les moyens de support et de guidage comprennent un palier lisse (15) délimité par un canon de guidage (7) prolongeant le boîtier (1) et une buse (22) prolongeant le générateur, traversée par la fibre et présentant, en direction du canon, une forme extérieure complémentaire au volume interne dudit canon.

9. Appareil selon l'une des revendications 5 ou 6, caractérisé en ce que les moyens d'entraînement sont constitués par au moins un élément souple (8) établi en boucle fermée, fixé au générateur (3) et entourant deux organes de renvoi (9) tournants, dont l'un est associé à l'organe de commande.

10. Appareil selon l'une des revendication 5 ou 6, caractérisé en ce que les moyens d'entraînement sont constitués par une noix (19) ou écrou solidaire du générateur et coopérant avec une vis sans fin (18) portée par le boîtier et associée à l'organe d'actionnement.

## Patentansprüche

1. Vorrichtung zur Anwendung von therapeutischen Behandlungen nach Art der Akupunktur, enthaltend:

— ein Gehäuse (1), das eine funktionelle Anordnung (2) aufnimmt, die durch einen Lichtstrahlengenerator (3) und eine optische Faser (5) gebildet ist, deren dem Generator entgegengesetzter Endteil aus dem Gehäuse ragt,

— eine Einrichtung (8-9) für das verschiebende Antreiben beim Ausfahrhub und/oder Rückzughub der Faser gegenüber dem Gehäuse,

— eine Einrichtung (12-13-15) für das Tragen und Führen beim Verstellen.

2. Vorrichtung nach Anspruch 1, dadurch gekennzeichnet, dass die Einrichtungen für das Antreiben (8), das Tragen (12) und das Führen (13-15) mit einer biegsamen optischen Faser (5) zusammenarbeiten, die sich ausgehend vom Generator (3) erstreckt und deren Länge grösser als der Abstand zwischen dem Generator und einem Anschlag (17) ist, der vom Gehäuse für die Anlage am Endstück (16) im zurückgezogenen Zustand der Faser gebildet ist.

3. Vorrichtung nach Anspruch 2, dadurch gekennzeichnet, dass die Einrichtung für das Antreiben durch ein bewegliches Organ gebildet ist, das die Überschusslänge der Faser (5) beaufschlagt und mit einem Steuerorgan verbunden ist.

4. Vorrichtung nach Anspruch 2, dadurch gekennzeichnet, dass die Einrichtungen für das Tragen und Führen wenigstens ein Lager (13 oder 15) aufweisen, das durch wenigstens ein Bauelement des Gehäuses gebildet ist.

5. Vorrichtung nach Anspruch 1, dadurch gekennzeichnet, dass die Einrichtungen für das Antreiben, Tragen und Führen mit einer aktiven Anordnung zusammenarbeiten.

6. Vorrichtung nach Anspruch 5, dadurch gekennzeichnet, dass die mit einem Steuerorgan verbundene Einrichtung (8) für das Antreiben die Verstellung des die Faser verschiebend antreibenden Generators (3) erzeugt, und dass die Einrichtungen für das Tragen und Führen aufweisen:

— Gleitführungen (12-10a) zwischen dem Gehäuse (1) und dem Generator (3),

— wenigstens ein Lager (13-15), das für das Tragen und Führen der Faser durch wenigstens ein Element des Gehäuses gebildet ist.

7. Vorrichtung nach einem der Ansprüche 4 oder 6, dadurch gekennzeichjnet, dass die Einrichtungen für das Tragen und Führen aufweisen: einglattes Lager (13), das durch eine Querwand (14) des Gehäuses gebildet ist, und ein glattes Rohr (15), das durch eine das Gehäuse verlängernde Führungshülse (7) gebildet ist.

8. Vorrichtung nach einem der Ansprüche 4 oder 6, dadurch gekennzeichnet, dass die Einrichtungen für das Tragen und Führen aufweisen: ein glattes Lager (15), das durch eine das Gehäuse verlängernde Führungshülse (7) gebildet ist, und eine Düse (22), die den Generator verlängert, von der Faser durchquert wird und in Richtung der Hülse eine zum Innenraum der Hülse komplementäre Aussenform aufweist.

9. Vorrichtung nach einem der Ansprüche 5 oder 6, dadurch gekennzeichnet, dass die Einrichtung für das Antreiben aus wenigstens einem biegsamen Element (8) besteht, das als geschlossener Kreislauf ausgelegt ist, am Generator (3) befestigt ist und zwei sich drehende Umlenkorgane (9) umgibt, von denen das eine mit dem Steuerorgan verbunden ist.

10. Vorrichtung nach einem der Ansprüche 5 oder 6, dadurch gekennzeichnet, dass die Einrichtung für das Antreiben durch eine Nuss (19) oder Mutter gebildet ist, die mit dem Generator fest verbunden ist und mit einer Schnecke (18) zusammenarbeitet, die vom Gehäuse getragen und mit dem Betätigungsorgan verbunden ist.

## Claims

1. Apparatus for the application of therapeutical treatment of the acupuncture type, comprising:

— a casing (1) containing a functional assembly (2) constituted by a generator of light radiation (3) and an optical fiber (5) the end part of which opposed to the generator protrudes from the casing,

— means (8, 9) of slidably driving the fiber according to an extension and/or retraction stroke with respect to the casing,

— supporting and guiding means (12, 13, 15) throughout displacement.

2. Apparatus according to Claim 1, characterized in that the driving (8), supporting (12) and guiding (13, 15) means cooperate with a flexible optical fiber (3) extending from the generator (5) and having a length greater than the distance separating said generator from a thrust (17) constituted by the casing against which rests the end piece (16) when the fiber is retracted.

3. Apparatus according to Claim 2, characterized in that the driving means are constituted by a movable member which takes over the excess length of the fiber (5), and is associated to a control member.

4. Apparatus according to Claim 2, characterized in that the supporting and guiding means at least comprise a bearing (13 or 15) provided on at least a constitutive element of the casing.

5. Apparatus according to Claim 1, characterized in that the driving, supporting and guiding means cooperate with the active assembly.

6. Apparatus according to Claim 5, characterized in that the driving means (8) associated to a control member, cause the displacement of the generator (3) carrying the fiber (5) in a sliding movement, and in that the supporting and guiding means comprise:
— sliding guides (12, 10a) provided between the casing (1) and the generator (3),
— at least a bearing (13, 15) provided by at least one element of the casing for supporting and guiding the fiber.

7. Apparatus according to Claim 4 or 6, characterized in that the supporting and guiding means comprise a smooth bearing (13) provided by a transversal wall (14) of the casing and a smooth bearing (15) defined by a guiding barrel (7) extending from the casing.

8. Apparatus according to Claim 4 or 6, characterized in that the supporting and guiding means comprise a smooth bearing (15) defined by a guiding barrel (7) extending from casing (1) and a nozzle (22) extending from the generator, traversed by the fiber and having, in the direction of the barrel, an external shape which is complementary to the internal volume of said barrel.

9. Apparatus according to Claim 5 or 6, characterized in that the driving means are constituted by at least a flexible element (8) set into a closed loop secured to the generator (3) and surrounding two rotary reversing members (9), one of which is associated to the control member.

10. Apparatus according to Claim 5 or 6, characterized in that the driving means are constituted by a sheath (19) or nut integral with the generator and cooperating with an endless screw (18) supported by the casing and associated to the actuating member.

**Fig. 1**

**Fig. 3**

**Fig. 2**

**Fig. 4**

0 074 914

Fig.7

Fig.6

Fig.5